(19)

Europäisches Patentamt
European Patent Office
Office européen des brevets

(11) **EP 2 584 338 A1**

(12) **EUROPEAN PATENT APPLICATION**

(43) Date of publication:
24.04.2013 Bulletin 2013/17

(51) Int Cl.:
*G01N 21/17* *(2006.01)*     *G01N 21/51* *(2006.01)*
*G01N 33/543* *(2006.01)*     *G01N 27/74* *(2006.01)*

(21) Application number: 11187706.4

(22) Date of filing: 03.11.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 19.10.2011  US 548852 P

(71) Applicant: Koninklijke Philips Electronics N.V.
5621 BA Eindhoven (NL)

(72) Inventor: **The designation of the inventor has not yet been filed**

(74) Representative: **Jungen, Carmen**
**Philips Intellectual Property &**
**Standards GmbH**
**Weißhausstraße 2**
**52066 Aachen (DE)**

(54) **Detection of clusters of magnetic particles**

(57)     The invention relates to the detection of clusters (C) comprising at least one magnetic particles (MP). The clusters (C) are moved by a time-variable magnetic field (B), and a detection signal (S) relating to this movement is generated and evaluated with respect to its temporal spectrum. The magnetic field (B) may in particular rotate with a basic frequency f, and the ratio between the signal components at this basic frequency f and/or higher harmonics may be determined. This ratio provides information about the distance between the magnetic particles (MP) in a cluster and thus allows to infer if the magnetic particles are nonspecifically bound or specifically bound via an intermediate target component.

Fig. 3

EP 2 584 338 A1

**Description**

FIELD OF THE INVENTION

The invention relates to a method and a device for the detection of clusters comprising at least one magnetic particle.

BACKGROUND OF THE INVENTION

The detection of clusters comprising magnetic particles by rotating them and detecting the light scattered in a dark field configuration has been described in literature (Ranzoni, A., Schleipen, J.J.H.B., van Ijzendoorn, L.J. & Prins, M.W.J., "Frequency-Selective Rotation of Two-Particle Nanoactuators for Rapid and Sensitive Detection of Biomolecules", Nano Lett 11, 2017-2022). In practical tests, this approach has shown good performances in complex matrixes with many interfering agents. In general, it is however difficult to discriminate between clusters having a similar structure, particularly between clusters in which two magnetic particles are directly ("nonspecifically") bound and clusters in which two magnetic particles are "specifically" bound via an intermediate target component.

SUMMARY OF THE INVENTION

It is therefore an object of the present invention to provide means that allow for an improved discrimination between different clusters comprising magnetic particles.

This object is achieved by a method according to claim 1 and a device according to claim 2. Preferred embodiments are disclosed in the dependent claims.

According to a first aspect, the invention relates to a method for the detection of at least one cluster in a sample, wherein the cluster comprises at least one magnetic particle. In this context, the term "magnetic particle" shall comprise both permanently magnetic particles as well as magnetizable particles, for example superparamagnetic beads. The size of the magnetic particles typically ranges between 3 nm and 50 $\mu$m. The "clusters" are agglomerates of two or more (magnetic or non-magnetic) particles which are coupled by some kind of binding. Of particular interest are specific (chemical) bindings via special chemical groups and intermediate components of interest and, in contrast thereto, nonspecific bindings that are e.g. merely caused by magnetic attraction forces between magnetized particles. The method comprises the following steps, which can be executed in the listed or any other appropriate order:

a) Exposing the at least one cluster to be detected to a time-variable magnetic field such that a movement of and/or in the cluster is induced by magnetic forces and/or torques that are exerted on the magnetic particle(s) within the cluster.

b) Generating a detection signal that is related to the aforementioned induced movement of the at least one cluster. The detection signal hence varies in dependence on the position, orientation, velocity, acceleration and/or another movement-related parameter of the one or more magnetic particles in the cluster. The detection signal will typically be something like a voltage, a current or a digital signal generated by a sensor.

c) Evaluating the aforementioned detection signal with respect to its (temporal) spectrum. This means that the (temporal) Fourier spectrum of the detection signal is determined at least for some frequencies such that further clues can be drawn therefrom.

According to a second aspect, the invention relates to a device for the detection of at least one cluster comprising at least one magnetic particle in a sample, said device comprising the following components:

a) A container in which the sample with the at least one cluster to be detected can be provided. The sample container may for example be realized as a disposable cartridge made from plastic by injection molding.

b) A magnetic field generator, comprising for example a permanent magnet or an electromagnet, for generating a time-variable magnetic field which induces movements of and/or in the cluster to be detected. Movements "in" the cluster are typically movements of its magnetic particle(s).

c) A detection unit for generating a detection signal that is related to the aforementioned induced movement of the at least one cluster.

d) An evaluation unit for evaluating the detection signal with respect to its spectrum. The evaluation unit may be realized by dedicated electronic hardware, digital

data processing hardware with associated software, or a mixture of both.

The method and the device are realizations of a common inventive concept, i.e. the spectral evaluation of a detection signal obtained from actuated clusters. Explanations and definitions provided for one of these realizations are therefore valid for the other realization, too.

As experiments have shown, the approach realized by the method and the device allows to gain information which may help to discriminate between clusters of similar, but slightly different, constitution. Surprisingly it is for example possible to distinguish between nonspecifically and specifically bound clusters of two magnetic particles by evaluating (a part of) the frequency content of the detection signal.

In the following, various preferred embodiments of the invention will be described that relate both to the method and the device described above.

The temporal variation of the magnetic field that acts on the clusters may in general be arbitrary. According to a preferred embodiment, the magnetic field is however varied periodically. The period of this field then provides a basic frequency which reappears in the detection signal. In typical examples, at least one component of the magnetic field may for example be a square wave or a sinusoidal wave.

According to another preferred embodiment of the invention, the magnetic field rotates at least partially (i.e. at least one component of the field vector rotates at least partially in a given plane, wherein a "partially rotating" time-dependent field has orientations in a limited angular space, e.g. in an angular segment of less than 360°). Thus a corresponding rotation of the clusters can be induced.

The spectral evaluation of the detection signal may particularly comprise the determination of the signal spectral components (Fourier coefficients) at a basic frequency and at least one higher harmonic thereof or at least two higher harmonics of the basic frequency.

If movement of the clusters is induced by a magnetic field that periodically varies (e.g. rotates) with a frequency f, this frequency may be taken as the aforementioned "basic frequency", and the signal spectral components may preferably be determined at $(n \cdot f)$ and/or $(2n \cdot f)$ and/or still higher harmonics $(kn \cdot f)$, where n and k are given natural numbers. Experiments suggest that n reflects the n-fold rotation symmetry of the rotating cluster. A two-particle cluster corresponds for example to n = 2, suggesting the observation of the signal spectral components $F_{2f}$, $F_{4f}$ etc. at 2f, 4f etc. If, for example, three particle clusters are considered, arranged in a perfect symmetrical triangle, the relevant signal spectral components would be $F_{3f}$, $F_{6f}$, $F_{9f}$, etc. at the frequencies 3f, 6f, 9f, etc. Furthermore, any asymmetry in the shape of a cluster and/or properties of the individual particles making up the cluster will result in also intermediate higher harmonics at 2f, 3f, 4f, 5f, 6f, 7f, etc.

According to a further development of the mentioned embodiments, the evaluation of the detection signal may comprise the determination of the ratio between the mentioned signal spectral components at at least two higher harmonics of the basic frequency. Experiments have for example shown that the ratio between the signal spectral components at 4f and 2f (with f being the basic frequency) comprises valuable information about two-particle clusters. In particular, the 4f signal seems to contain information about the gap size whereas the 2f signal does not. Use of a ratio is preferable because it is independent of the number of clusters in the sample (which shows variations) and is therefore more accurately describing the properties of the sample.

In general, the detection signal may originate from any detection modality. It may for example be determined from magnetic effects induced by the actuated clusters. In a preferred embodiment, the detection signal originates from an optical detection. A light detector may then be provided for detecting output light that has interacted with the clusters, particularly output light that has been scattered by the clusters. The light detector may for example comprise photodiodes, photo resistors, photocells, a CCD chip, or a photo multiplier tube.

In the aforementioned embodiment, the scattered output light may originate from ambient light. In a preferred embodiment, a technical light source is however provided for emitting input light into the sample. The illumination can then more precisely be controlled. If the clusters to be detected are rotated, the input light may in general come from any direction with respect to the plane of rotation, e.g. perpendicular. Preferably, the input light will however propagate in the plane of rotation.

In an embodiment in which both the above-mentioned light detector and light source are used, the light detector is preferably disposed oblique with respect to the direction of the input light, i.e. at an angle (strictly) between 0° and 180°. Most preferably, an angle of roughly 45° is used (i.e. an angle between about 20° and 60°).

The clusters to be detected preferably comprise (at least) two particles each (at least one of which is a magnetic particle). Thus a symmetrical configuration is provided for which a rotational movement can be observed.

In the aforementioned embodiment, the two particles may be specifically bound via an intermediate target component. The specificity of this binding is typically used to detect particular target components of interest in a sample by linking them to the (magnetic) particles in a cluster. The target component can for example be a biomolecule, which can have a size between a nanometer and several tens of nanometers; or it can be a composite containing several biomolecules; or it can be a larger moiety such as a virus or a small organism; or it can be material derived from a biological cell or from tissue, e.g. material derived from cellular membrane or from the intercellular and/or extracellular space, or e.g. microparticles that are shed by cells."

Scattering and subsequent interference of light, originating from the rotating cluster, leads to maxima and minima in the scattered intensity as a function of the orientation of the cluster with respect to the incoming optical field. It can be shown that this scattering behavior is strongly dependent on the distance between the two particles of a two-particle cluster. In case of such clusters, the spectral evaluation of the detection signal is hence preferably applied to infer the distance between these particles and/or to infer the presence or absence of an intermediate target component of the aforementioned kind. The possibility to discriminate between clusters with and without an intermediate target component allows to increase the detection accuracy as specifically bound clusters (target signal) can be distinguished from nonspecifically bound clusters (background). Alternatively, the distance between the particles can be used as a measure for biomarker binding, by including molecular switches between the particles to which the biomarker can bind, wherein switches with or without biomarker yield different conformations and different inter-particle distances (cf. Maye et al., "Switching binary states of nanoparticle superlattices and dimer clusters by DNA strands", Nature Nanotechnology vol. 5, p. 116 (2010)).

The observed spectrum of the detection signal will usually depend on the time course of the magnetic field with which movement of the clusters is induced. Accordingly, the temporal course of the magnetic field is preferably optimized such that a high correlation between the spectral characteristics of the detection signal and a parameter of interest is achieved, for example the size of the one or more clusters, or the inter-particle distance, or the nature of the inter-particle bond, or the number of molecular bonds between the particles, or the nature of the molecular architecture on the particle, or the magnetic or optical properties of the particle, or the shape of the particle, or the viscosity or other mechanical properties of the sample. If the temporal course of the magnetic field is determined by a basic frequency, the optimization may for example comprise the determination of an optimal value of this frequency.

BRIEF DESCRIPTION OF THE DRAWINGS

These and other aspects of the invention will be apparent from and elucidated with reference to the embodiments described hereinafter.

In the drawings:

Fig. 1    schematically sketches how magnetic particles bind via a target molecule to a cluster;

Fig. 2    is an enlarged picture of a sandwich configuration of two magnetic particles and one target molecule;

Fig. 3    schematically sketches a device according to the present invention;

Fig. 4    is a diagram representing the time course of a recorded optical detection signal when the magnetic field is rotating at 1 Hz;

Fig. 5    shows the frequency spectrum of the signal of Figure 4;

Fig. 6    shows the average hydrodynamic radius for 500 nm magnetic particles with different surface architectures;

Fig. 7    shows the ratios of the 4th and 2nd harmonics of the detection signal for clusters formed from the different surface architectures of Fig. 6;

Fig. 8    shows the ratios of the 4th and 2nd harmonics for a blank measurement (no bBSA, right) and for low concentrations ofbBSA when only doublets are present (left);

Fig. 9    shows the ratios of the 4th and 2nd harmonics for clusters formed via PSA from the COOH and multilayer molecular architecture;

Fig. 10    shows the frequency dependence of higher harmonics of the detection signal.

Like reference numbers refer in the Figures to identical or similar components.

DETAILED DESCRIPTION OF EMBODIMENTS

In recent years, an increased demand for more pervasive and effective healthcare systems is affecting the world of in vitro diagnostics, putting the stress on the achievement of effective point-of-care solutions. The achievement of such goal is particularly demanding due to the requirements of point-of-care diagnosis: since the tests need to be performed at the patient location, they must be rapid, sensitive, quantitative and accurate. Moreover the platform on which the test is performed needs to be portable and easy-to-use.

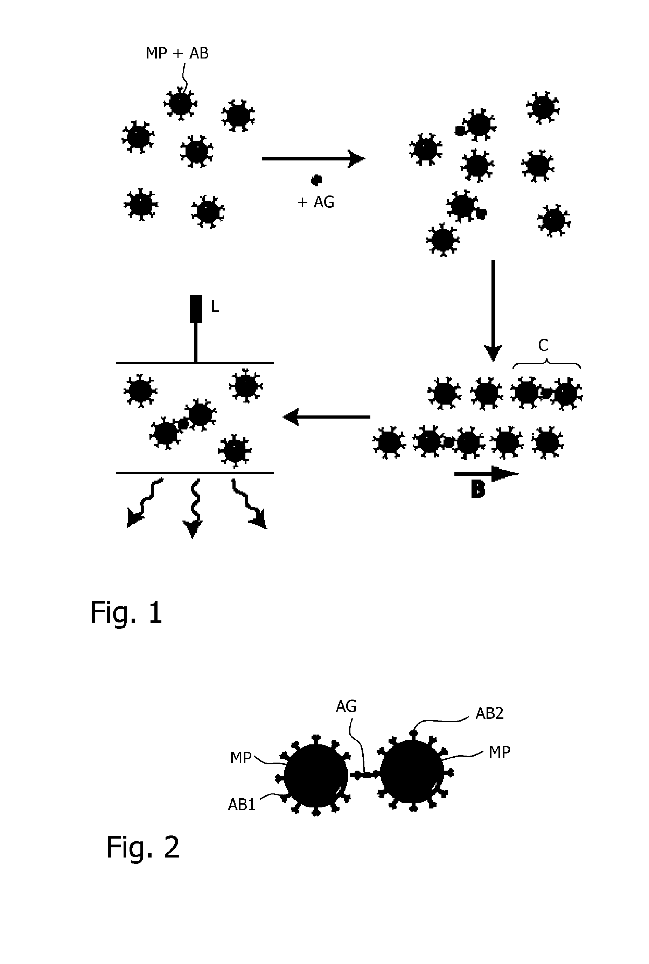

Magnetic cluster assays provide a volumetric and surface-free architecture and therefore they are intrinsically rapid and cost-effective. Figure 1 schematically sketches four consecutive steps of a typical magnetic cluster assay based on magnetic confinement of the magnetic (e.g. nano-) particles MP to enable effective cluster formation.

As a first step (Figure 1 top left), the magnetic particles MP coated with antibodies are provided. In a second step (Figure 1 top right), the antigen AG is added and binds to magnetic particles. In a third step (Figure 1 bottom right), the sample is exposed to a magnetic field B, causing the formation of particle chains or clusters C. In the last step (Figure 1 bottom left), the sample is illuminated with a light source L, and the light scattered by the clusters is detected.

Different agglomeration assay formats can be used, e.g. a sandwich assay or a competition assay. As an example, Figure 2 illustrates a particular sandwich assay format where the magnetic particles MP are coated with different monoclonal antibodies AB1 and AB2. The antibodies can specifically bind different sites of the antigen AG, thus forming a two-particle cluster with a sandwich configuration.

Good sensitivities and low limit of detection in the picomolar range have been demonstrated for such approaches (Baudry et al., Proceedings of the National Academy of Sciences 2006, 103, (44), 16076.). Essentially, such a step enables effective cluster formation by up-concentrating the magnetic particles and forcing them to remain in close contact between each other despite their negative surface charge. A typical magnetic actuation protocol consists for example in applying a uniform magnetic field to a sample containing magnetic particles that have been incubated with the target biomolecule to detect. When the field is active, the particles arrange themselves into chains and are free to vibrate and rotate while in close proximity with each others. Consequently the specific bond can be effectively formed.

Moreover, an innovative detection principle has been introduced, where the formed clusters are quantified by rotating them and detecting the light scattered in dark field configuration (Ranzoni, A., Schleipen, J.J.H.B., van Ijzendoorn, L.J. & Prins, M.W.J., above). The novelty of the detection protocol lies in applying rotational magnetic actuation during the detection itself, which allows getting for example information about the size of the population of formed clusters, about binding efficiency and kinetics of the reaction. This assay demonstrates very good performances in complex matrixes, with high protein content and rich of endogenous interfering agents.

Low detection limits are an important challenge in in-vitro diagnostics, including point-of-care applications. Other challenges are speed, dynamic range, precision, etc. The current performances of cluster immunoassays are often limited by the non-specific binding: in the low-concentration limit only target-bound doublets (i.e. clusters comprising two magnetic particles) are present in solution, together with an undefined number of nonspecifically bound doublets. Both species generate a similar optical signal that, up to now, was undistinguishable.

In order to address this problem, a method is proposed here to further increase the robustness and the sensitivity of the assay by discriminating the specificity of the binding by means of harmonic analysis of the measured (particularly optical) signal.

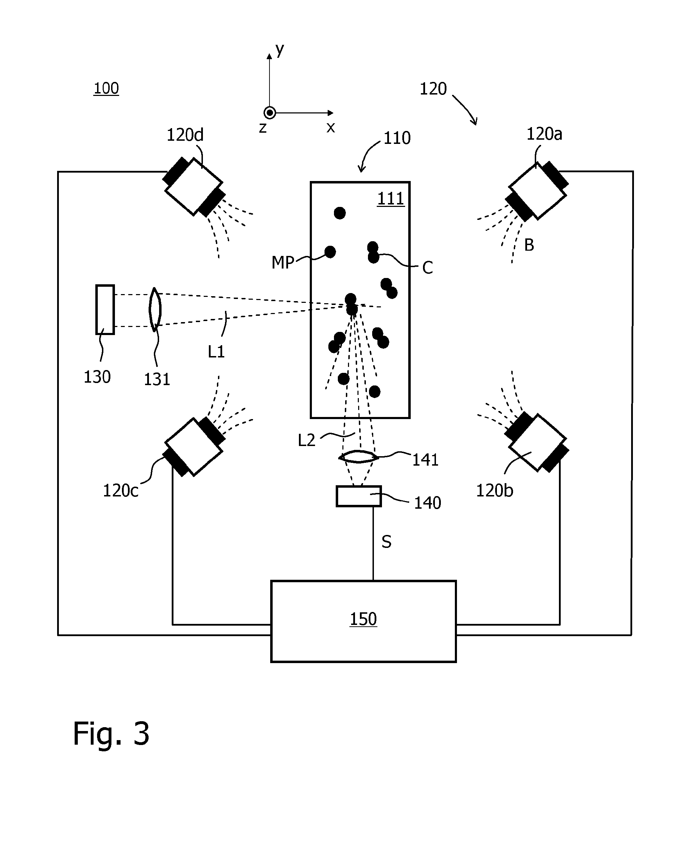

The approach proposed by the present invention can be implemented in a sensor device 100 (optomagnetic platform) as sketched in Figure 3. This sensor device 100 comprises a sample container or cartridge 110 with a sample volume 111 in which a sample fluid with magnetic particles or beads MP can be provided. In the shown example, the sample cartridge 110 is a glass tube of square cross section.

The sensor device 100 further comprises a reader, of which only the most relevant components are schematically sketched. These components comprise:

- A magnetic field generator 120, which in the shown example consists of four electromagnets 120a, 120b, 120c, 120d. These magnets are arranged at the corners of a rectangle or square and are aligned with their axes towards some centre within the sample volume 111. By an appropriate sequential control of the currents in the electromagnets, a uniform rotating magnetic field B can be generated within the sample volume that induces rotation of magnetic clusters C in the vertical plane. The field vector B may for example be described by a sinusoidal temporal variation according to

$$\underline{B} = A \cdot \begin{pmatrix} \sin(2\pi f t) \\ \cos(2\pi f t) \\ 0 \end{pmatrix}$$

with A being a constant, f being the rotation frequency, and the z-axis perpendicular to the plane of Figure 3.

- A light source 130, for example a laser diode or an LED, which emits a collimated (laser) beam of input light L1 that is focused by a lens 131 into the sample volume 111. The light source 130 is arranged in the (xy-) plane in which the magnetic field B and the clusters C rotate. Arrangements oblique — particularly perpendicular — to this plane are possible, too.

- A light detector 140, for example a photodiode or an image sensor.
  Output light L2 coming from the sample volume 111 is focused by means of a lens 141 onto the active surface of the light detector. The output light L2 consists in the shown embodiment of input light L1 that was scattered by components of the sample volume, particularly by magnetic particles MP and/or clusters C of these particles. The light detector 140 is arranged in this embodiment at a "detection angle" of about 90° with respect to the input light L1 (in practice, smaller detection angles of about 45° will typically be preferred).

- A control and evaluation unit 150 that is coupled to the magnetic field generator 120 and to the light detector 140 in order to control them and to process the detection signals S provided by the light detector 140.

While rotating, the clusters C in the sample volume 111 expose a time-dependent cross-section to the incoming light L1, therefore introducing a modulation of the light intensity scattered by the sample in the output light L2. The resulting detection signal S is forwarded to the control and evaluation unit 150. A FFT (Fast Fourier Transform) algorithm can be applied there to the recorded signal S.

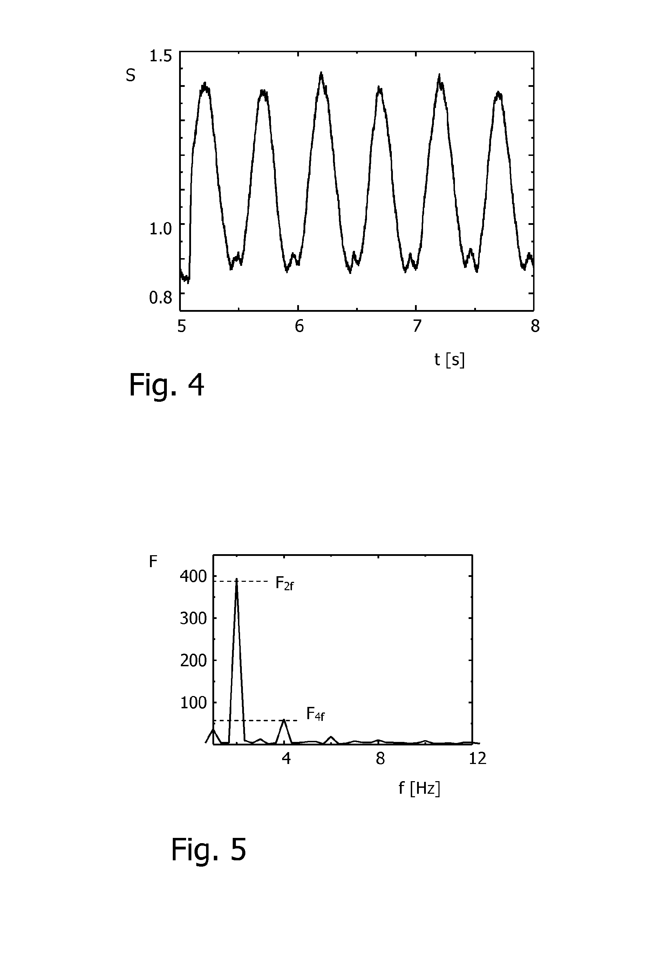

Figure 4 shows a typical dependency of the optical signal S (arbitrary units) on time t when the magnetic field is rotating at f = 1 Hz. It is possible to see that the signal is mainly at 2 Hz, but also presents higher harmonics.

Figure 5 shows the Fourier Transform of the curve of Figure 4. The frequency components and the magnitude of the higher harmonics of the optical signal can be seen.

Because of their uniaxial symmetry, the (two-particle) clusters (doublets) are exposing the same cross-section to the incoming light beam L1 twice per period, therefore generating a modulation of the scattered light L2 at double the frequency f of the magnetic field B. Because of interference and non-linear effects, such modulation also contains features at higher frequencies, as visible in the Fourier spectrum of Figure 5.

In the following discussion, the amplitude $F_{2f}$ of the FFT at twice the basic frequency f of the magnetic field is defined as signal, since it showed up as the strongest of all the harmonics. Surprisingly it was found that the magnitude $F_{4f}$ of the harmonic at four times the applied frequency (i.e. 4f) relates to the number of clusters as well as to the gap size between two magnetic particles in a cluster C. For this reason, the ratio of the signal amplitudes $F_{4f}$ and $F_{2f}$ at four times and two times the basic frequency f, respectively, i.e. the value

$$R_{4f/2f} = F_{4f} / F_{2f}$$

will below be used as a characteristic parameter derived from this spectrum. The mentioned result was derived as follows:

Several MP surface molecular architectures were engineered, which result in an increased spacer layer on top of the corresponding (single) magnetic nanoparticles MP.

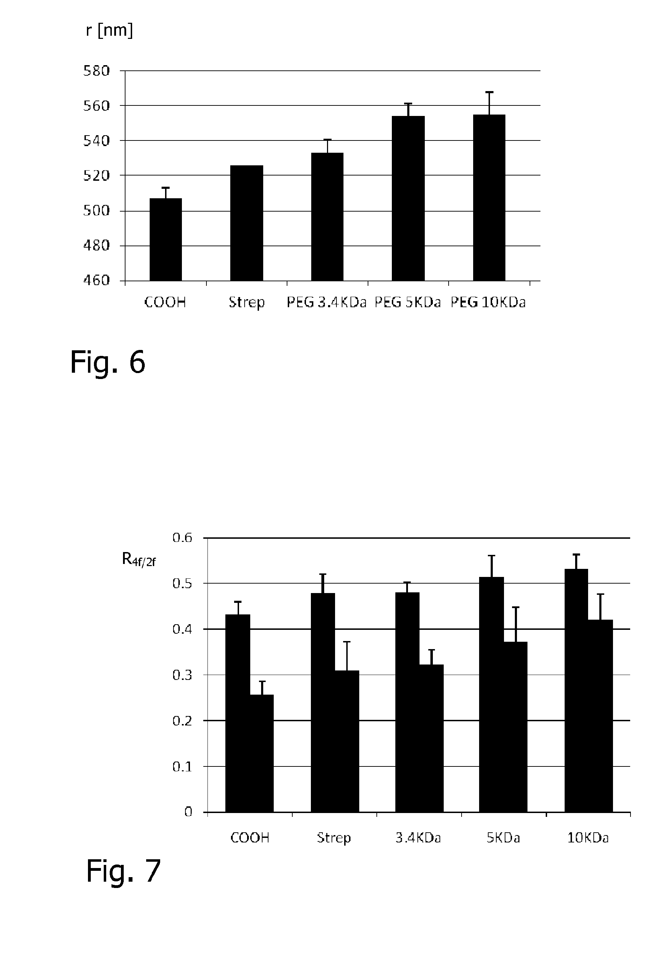

The hydrodynamic radius r of these particles was measured by dynamic light scattering. It is plotted in Figure 6 for the investigated architectures. The first architecture is carboxyl-terminated (COOH) groups, the second is streptavidin (Strep) coating, the third, fourth and fifth are composed of PEG linkers of respectively 3.4 KDa, 5 KDa and 10 KDa of molecular weight.

Figure 7 shows the ratio $R_{4f/2f}$ of 4th and 2nd harmonics for the different surface architectures described in Figure 6. The diagram contains the results from ten independent experiments (wavelength 658 nm, intensity 18 mW, p polarized) for two different detection angles of 45° (left bars) and 30° (right bars). It is possible to see from this a proportional relationship: for increasing gap size the ratio $R_{4f/2f}$ also increases.

This feature may be attributed to the fact that each nanoparticle scatters light mostly in the forward direction with respect to the optical axis, therefore shining light into the second nanoparticle when the cluster is aligned with the optical beam. Such injection of scattered light generates a stronger scattering from the second nanoparticle, which is responsible for the local smaller maximum contributing the most to the 4th harmonic. Also the effect seems to be most pronounced when detecting the scattered light L2 at about 45° orientation with respect to the exciting laser beam L1.

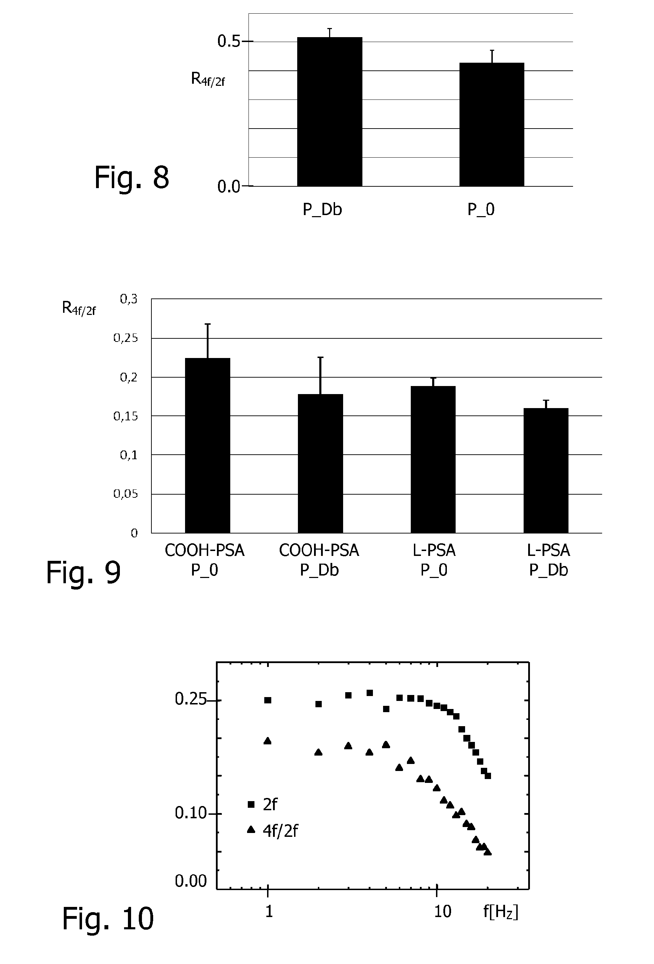

When running an immunoassay, it is important to appreciate the presence or absence of a target component, for example the biomarker antigen AG in the setup of Figure 2. The physical dimensions of such a target component are typically of the order of a few nm. As a test of this possibility, a large biomolecule (biotinylated BSA or bBSA) was used in an assay with streptavidin coated magnetic nanoparticles. Due to the strong dependence of the scattering and subsequent interference of light on the gap size, such small difference in distances could be appreciated, as shown in Figure 8. This Figure shows the ratio $R_{4f/2f}$ of 4th to 2nd harmonic for twelve blank measurements P_0 (no bB SA) and for twelve low concentration measurements P_Db at concentrations of bBSA biomolecules low enough that only doublets are present in the sample (the critical frequency — i.e. the maximum frequency at which the cluster can rotate synchronously with the field — relates to the size of the clusters in solution and was monitored as a function of the target concentration). In the latter case, the sample is characterized by a mixture of specific and non-specific doublets in the midst of a large number of single magnetic nanoparticles. When inspecting the graph it is possible to see that the ratio of the harmonics differs with statistical significance, indicating that the increase in gap size influences the optical signal.

Furthermore, the behavior of a biomarker for prostate cancer (Prostate Specific Antigen or PSA) was analyzed for two different bead surface molecular architectures. First, a more conventional surface chemistry was used, where the antibodies against PSA are coupled directly to the surface of carboxyl-terminated nanoparticles by EDC (1-ethyl-3-(3-dimethylaminopropyl) carbodiimide) chemistry. Such coupling is not optimal for the assay, since the antibodies are not oriented with respect to the nanoparticles and therefore can exhibit limited activity.

Figure 9 shows the ratio $R_{4f/2f}$ of the harmonics, where "COOH-PSA P_0" stands for the blank solution without PSA and "COOH-PSA P_Db" stands for the solution with PSA in which doublets (two-particle clusters) are formed. In case of COOH chemistry however, the low reproducibility translates in low statistical relevance. The low reproducibility may be attributed to the fact that successful binding can occur only if the target is captured by an antibody which is sterically accessible by a neighbor nanoparticle. Given the high surface roughness, the gap size can exhibit large variations, which reflects in variations in the ratio of harmonics.

The aforementioned results were benchmarked with respect to a multi-layer architecture based on PEG linkers. Figure 9 shows the results for ten measurements for blanks ("L-PSA P_0") and for concentrations of PSA that result in only doublets ("L-PSA P_Db"). The multilayer architecture results in a very reproducible assay and well defined gap size, leading to a change in the ratio of harmonics between blanks and doublet regime which is statistically relevant. Such technique can be exploited to boost the limit of detection and discriminate the presence of biomarker at extremely low concentrations, where the signal approaches the blank value, conferring robustness to the immunoassay.

Another feature of the invention lies in analyzing the ratio $R_{4f/2f}$ as a function of frequency f. By increasing the frequency f of the applied magnetic field, the shear stress experienced by each magnetic particle increases, therefore transmitting a force to the biomolecule in between. In particular, during one period of rotation the dipole-dipole interaction is always attractive and mild (< 1 pN) and it is consequently not very suited to probe molecular bonds. Hydrodynamic forces can be accurately controlled by the magnetically-induced rotational behavior, resulting in forces of the order of few pN, relevant to probe the biophysical properties of many biomolecules (i.e. DNA, polymer linkers etc.). To prove this, experiments were performed by measuring the frequency response of multilayered coated nanoparticles.

The results of these experiments are represented in Figure 10, which shows the frequency dependence of the higher harmonics (the squares "2f" representing the normalized component of the spectrum at 2f, the triangles "4f/2f" representing the ratio of the frequency components at 4f and 2f). When rotating at increasing frequency f, the nanoparticles move progressively apart and an increase of the relative distance translates in a decreased ratio $R_{4f/2f}$ (see corner point at approximately 7 Hz in Figure 10). Between 7 Hz and 12 Hz, the doublets are still rotating synchronously with the field (see the graph of the normalized 2f values in Figure 10).

In summary, a procedure was described in which nanometer sensitivity in the distance between nanoparticles can be achieved by accurately monitoring the spectral content of the signal. The distance determination is for example relevant in making a distinction between specific and non-specific binding, which increases the sensitivity in the low concentration limit of biosensing.

The detection volume of a biosensor can have different sizes with respect to the sample volume. For example, the detection volume can be as large as the total sample volume, but the detection volume can also be much smaller. An advantage of having a small detection volume is that the number of magnetic particles and/or clusters in the detection volume will be low. For example, even single particles and/or single clusters can be resolved in the detection signal. Having a small detection volume will facilitate the detection of small differences between particles and/or clusters. E.g. differences between specific versus non-specific binding. A disadvantage of having a very small detection volume is that the number of magnetic particles and/or clusters may be so low that number fluctuations give an imprecise measurement outcome. In order to improve statistics, the detection volume may be scanned through the sample, or magnetic particles and/or clusters may be scanned through the detection volume. The scanning of magnetic particles and/or clusters through the detection volume may e.g. be realized by flowing the fluid (e.g. as in a flow cytometer or in a microfluidic device) and/or by using magnetic forces.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method for the detection of at least one cluster (C) comprising a magnetic particle (MP) in a sample, said method comprising the following steps:

   a) exposing the at least one cluster (C) to a time-variable magnetic field (B) that induces a movement of and/or in the cluster (C);
   b) generating a detection signal (S) that is related to said induced movement;
   c) evaluating the detection signal (S) with respect to its spectrum.

2. A device (100) for the detection of at least one cluster (C) comprising a magnetic particle (MP) in a sample, comprising:

   a) a sample container (110) in which the sample can be provided;
   b) a magnetic field generator (120) for generating a time-variable magnetic field (B) that induces a movement of and/or in the at least one cluster (C);
   c) a detection unit (140) for generating a detection signal (S) that is related to said induced movement;
   d) an evaluation unit (150) for evaluating the detection signal (S) with respect to its spectrum.

3. The method according to claim 1 or the device (100) according to claim 2, **characterized in that** the magnetic field (B) varies periodically.

4. The method according to claim 1 or the device (100) according to claim 2, **characterized in that** the magnetic field (B) rotates at least partially.

5. The method according to claim 1 or the device (100) according to claim 2, **characterized in that** the evaluation of the detection signal (S) comprises the determination of the signal spectral component ($F_{2f}$, $F_{4f}$) at a basic frequency (f) and/or higher harmonics thereof.

6. The method or the device (100) according to claim 5, **characterized in that** the basic frequency corresponds to a variation frequency f of the magnetic field (B) and that the signal spectral components ($F_{2f}$, $F_{4f}$) are determined at $n \cdot f$ and/or $2n \cdot f$, and optionally higher harmonics, where n reflects the n-fold rotation symmetry of the rotating cluster (C).

7. The method or the device (100) according to claim 5, **characterized in that** the evaluation of the detection signal (S) comprises the determination of the ratio ($R_{4f/2f}$) of two of said signal spectral components ($F_{2f}$, $F_{4f}$).

8. The method according to claim 1 or the device (100) according to claim 2, **characterized in that** a light detector (140) is provided for detecting output light (L2) that has interacted with the at least one cluster (C), preferably output light (L2) that has been scattered by the at least one cluster (C).

9. The method according to claim 1 or the device (100) according to claim 2, **characterized in that** a light source (130) is provided for emitting input light (L1) into the sample.

10. The method or the device (100) according to claim 8 and 9,
**characterized in that** the light detector (140) is disposed oblique with respect to the direction of the input light (L 1).

11. The method according to claim 1 or the device (100) according to claim 2, **characterized in that** the at least one cluster (C) comprises two particles (MP).

12. The method or the device (100) according to claim 11,
**characterized in that** the two particles (MP) may be specifically bound via an intermediate target component (AG).

13. The method or the device (100) according to claim 11 or 12, **characterized in that** the distance between the particles (MP) and/or the presence or absence of a target component (AG) is inferred from the spectral evaluation of the detection signal (S).

14. The method according to claim 1 or the device (100) according to claim 2, **characterized in that** the temporal course of the magnetic field (B) is optimized with respect to a high correlation between the spectral characteristics of the detection signal (S) and the properties of the at least one cluster (C).

MP + AB

+ AG

L

C

B

## Fig. 1

AG

AB2

MP

MP

AB1

## Fig. 2

Fig. 3

Fig. 4

Fig. 5

**Fig. 6**

**Fig. 7**

Fig. 8

Fig. 9

Fig. 10

Europäisches
Patentamt

European
Patent Office

Office européen
des brevets

# EUROPEAN SEARCH REPORT

Application Number

EP 11 18 7706

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/021142 A1 (KONINKL PHILIPS ELECTRONICS NV [NL]; RANZONI ANDREA [NL]; OVSYANKO MIK) 24 February 2011 (2011-02-24) * abstract; page 1, line 23 to page 6, line 20; page 8, lines 25-29; page 9, line 31 to page 12, line 28; figures 1,2 * | 1-4,8,9, 11-14 | INV. G01N21/17 G01N21/51 G01N33/543 G01N27/74 |
| X | WO 2010/026551 A1 (KONINKL PHILIPS ELECTRONICS NV [NL]; RANZONI ANDREA [NL]; PRINS MENNO) 11 March 2010 (2010-03-11) * abstract; page 1, lines 11-14; page 2, lines 15-18; page 3, line 5 to page 5, line 5 * | 1-4,8,9 | |
| X | VUPPU ANIL ET AL: "Phase sensitive enhancement for biochemical detection using rotating paramagnetic particle chains", JOURNAL OF APPLIED PHYSICS, AMERICAN INSTITUTE OF PHYSICS. NEW YORK, US, vol. 96, no. 11, 1 January 2004 (2004-01-01), pages 6831-6838, XP012068383, ISSN: 0021-8979, DOI: 10.1063/1.1809269 * abstract; Fig. 6 and associated text passages * | 1-6,8-10 | |
| X | US 4 725 140 A (MUSHA TOSHIMITSU [JP]) 16 February 1988 (1988-02-16) * abstract; col. 3, lines 4-68; col. 4, lines 46-57; col. 8, lines 37-58; figure 1 * | 1-6,8,9 | TECHNICAL FIELDS SEARCHED (IPC) G01N |

-/--

The present search report has been drawn up for all claims

| Place of search | Date of completion of the search | Examiner |
|---|---|---|
| Munich | 30 November 2012 | Weinberger, Thorsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 11 18 7706 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/126903 A1 (GARCIA ANTONIO A [US] ET AL) 1 July 2004 (2004-07-01)<br>* abstract;<br>paragraphs [0020], [0024], [0037] *<br>----- | 1-4,8,9 | |
| X | WO 2009/037636 A1 (KONINKL PHILIPS ELECTRONICS NV [NL]; PRINS MENNO WILLEM JOSE [NL]; IJZ) 26 March 2009 (2009-03-26)<br>* abstract; page 10, line 11 to page 11, line 32; page 13, line 1 to page 14, line 10; page 17, lines 15-24; page 21, lines 19-20;<br>figures 1,4 *<br>----- | 1-6,8,9, 11,13,14 | |
| X | EP 1 304 542 A2 (PHILIPS CORP INTELLECTUAL PTY [DE]; KONINKL PHILIPS ELECTRONICS NV [NL]) 23 April 2003 (2003-04-23)<br>* abstract;;<br>paragraphs [0008], [0013] - [0015], [0026], [0031]; figure 2 *<br>----- | 2,3 | |
| | | | **TECHNICAL FIELDS SEARCHED (IPC)** |

The present search report has been drawn up for all claims

| Place of search | Date of completion of the search | Examiner |
|---|---|---|
| Munich | 30 November 2012 | Weinberger, Thorsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 18 7706

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2012

| Patent document cited in search report | | Publication date | Patent family member(s) | | Publication date |
|---|---|---|---|---|---|
| WO 2011021142 | A1 | 24-02-2011 | CN | 102472750 A | 23-05-2012 |
| | | | EP | 2467722 A1 | 27-06-2012 |
| | | | US | 2012208296 A1 | 16-08-2012 |
| | | | WO | 2011021142 A1 | 24-02-2011 |
| WO 2010026551 | A1 | 11-03-2010 | CN | 102144162 A | 03-08-2011 |
| | | | EP | 2326956 A1 | 01-06-2011 |
| | | | JP | 2012502271 A | 26-01-2012 |
| | | | RU | 2011112853 A | 10-10-2012 |
| | | | US | 2011156701 A1 | 30-06-2011 |
| | | | WO | 2010026551 A1 | 11-03-2010 |
| US 4725140 | A | 16-02-1988 | DE | 3639352 A1 | 25-06-1987 |
| | | | JP | 1798610 C | 12-11-1993 |
| | | | JP | 5004021 B | 19-01-1993 |
| | | | JP | 62118255 A | 29-05-1987 |
| | | | US | 4725140 A | 16-02-1988 |
| US 2004126903 | A1 | 01-07-2004 | AU | 2003263986 A1 | 23-02-2004 |
| | | | US | 2004126903 A1 | 01-07-2004 |
| | | | WO | 2004012836 A2 | 12-02-2004 |
| WO 2009037636 | A1 | 26-03-2009 | CN | 101802614 A | 11-08-2010 |
| | | | EP | 2193375 A1 | 09-06-2010 |
| | | | US | 2012119727 A1 | 17-05-2012 |
| | | | WO | 2009037636 A1 | 26-03-2009 |
| EP 1304542 | A2 | 23-04-2003 | CN | 1412550 A | 23-04-2003 |
| | | | DE | 10151778 A1 | 08-05-2003 |
| | | | EP | 1304542 A2 | 23-04-2003 |
| | | | JP | 4335515 B2 | 30-09-2009 |
| | | | JP | 2003199767 A | 15-07-2003 |
| | | | US | 2003085703 A1 | 08-05-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RANZONI, A. ; SCHLEIPEN, J.J.H.B. ; VAN IJZEN-DOORN, L.J. ; PRINS, M.W.J.** Frequency-Selective Rotation of Two-Particle Nanoactuators for Rapid and Sensitive Detection of Biomolecules. *Nano Lett,* vol. 11, 2017-2022
- **MAYE et al.** Switching binary states of nanoparticle superlattices and dimer clusters by DNA strands. *Nature Nanotechnology,* 2010, vol. 5, 116
- **BAUDRY et al.** *Proceedings of the National Academy of Sciences,* 2006, vol. 103 (44), 16076